# FASCICULE DE BREVET EUROPEEN

(11) **EP 0 313 465 B1**
(45) Date de publication et mention de la délivrance du brevet: **28.04.1993**
(21) Numéro de dépôt: 88402647.7
(22) Date de dépôt: 20.10.1988
(51) Int. Cl.: C12P 7/02, C12P 7/04, C12G 1/06, A23C 9/12, A23L 1/226

(54) **Procédé d'obtention d'arômes terpéniques par un procédé microbiologique**
Verfahren zur Gewinnung von terpenischen Aromen durch ein mikrobiologisches Verfahren
Process for obtaining terpenic aromas by a microbiological process

(30) Priorité: 22.10.1987 FR 8714609
(43) Date de publication de la demande: 26.04.1989
(73) Titulaire: PERNOD-RICARD, F-75008 Paris (FR)
(72) Inventeur: Karst, Francis, F-86000 Poitiers (FR); Vladescu, Barbu Dinu Vladimir, F-75016 Paris (FR)
(74) Mandataire: Warcoin, Jacques

(56) Documents cités:
- CHEMICAL ABSTRACTS, vol. 102, no. 9, 4 mars 1985, page 466, résumé no. 77215f, Columbus, Ohio, US; R. HOCK et al.: "Formation of terpenes by yeast during alcoholic fermentation", & Z. LEBENSM.-UNTERS. FORSCH. 1984, 179(6),450-2
- CHEMICAL ABSTRACTS, vol. 95, no. 13, 28 septembre 1981, page 515, résumé no. 113321k, Columbus, Ohio, US; G.L. FAGAN et al.: "Production of linalool, cis- and trans-nerolidol, and trans, trans-farnesol by Saccharomyces fermentati growing as a film on simulated wine", & VITIS 1981, 20(1), 36-42
- J. AGRIC. FOOD CHEM., vol. 26, no. 3, 1978, pages 765-766, American Chemical Society; F. DRAWERT et al.: "Biosynthesis of flavor compounds by microorganisms. 3. Production of monoterpenes by the yeast kluyveromyces lactis"
- CHEMICAL ABSTRACTS, vol. 100, no. 15, 9 avril 1984, page 446, résumé no. 119290t, Columbus, Ohio, US; SH. A. ABRAMOV et al.: "Effect of Daghestan yeasts of the Saccharomyces genus on the composition of champagne aroma components", & PRIKL. BIOKHIM. MIKROBIOL. 1984, 20(1), 129-32
- CHEMICAL ABSTRACTS, vol. 98, no. 25, 20 juin 1983, page 433, résumé no. 214076b, Columbus, Ohio, US; A.K. RODOPULO et al.: "Effect of crushed wine yeast on champagne quality", & BIOKHIM. MIKROBIOL. 1983, 19(2), 292-6
- "Formation of terpenes by yeast during alcoholic fermentation", Z. Lebensm.-unters. Forsch., 1984, 179(6), 450-2

## Description

La présente invention concerne un procédé d'obtention d'arômes terpéniques, notamment pour l'aromatisation de boissons, par un procédé microbiologique mettant en oeuvre des mutants de Saccharomyces cerevisiae.

Les terpènes, considérés essentiellement comme produits d'origine végétale, sont des composés caractéristiques de nombreuses huiles essentielles. Grâce à leurs propriétés physiologiques et sensorielles, les mono et sesquiterpènes ont des applications multiples dans l'industrie alimentaire, dans la parfumerie et dans l'industrie pharmaceutique.

Par ailleurs, certains monoterpènes (géraniol, nérol, linalol, citronellol, alpha-terpinéol) sont responsables du caractère aromatique du raisin Muscat. Les variétés de raisin à goût et arôme Muscat appartiennent à deux classes distinctes, définies principalement par la valeur du rapport linalol/géraniol. Pour les variétés "Muscat", le rapport linalol/géraniol est supérieur à 1 tandis que pour les variétés "Malvoisie", il est nettement inférieur à 1).

Les sources végétales de monoterpènes, bien que nombreuses, pourraient, pour des raisons climatiques, saisonnières, économiques et politiques, s'avérer insuffisantes face à un marché de plus en plus demandeur, c'est pourquoi la production microbienne de ces principes aromatiques présente un intérêt considérable, c'est l'un des buts de la présente invention.

Contrairement aux champignons filamenteux, dont l'aptitude à synthétiser des terpènes a été souvent signalée, les levures ne semblent pourvues que faiblement de ces capacités biosynthétiques.

L'article de Z. Lebensm. Unter. Forsch. (1984), 179, 450-452, décrit la formation de traces de farnésol par S. cerevisiae.

La faible production de terpènes par les levures, et plus particulièrement par S. cerevisiae, est en apparente contradiction avec la présence dans les cellules de systèmes enzymatiques susceptibles de catalyser la biosynthèse de certains monoterpènes.

En effet, les cellules de S. cerevisiae synthétisent des quantités significatives (0,5 - 3 % du poids sec) d'ergostérol à partir de l'acétyl-CoA et la polymérisation de l'acétyl-CoA, comme chez les plantes supérieures, se fait via le mévalonate, le géranylpyrophosphate et le farnésylpyrophosphate. S. cerevisiae possède donc toutes les enzymes nécessaires à la biosynthèse des terpènes et, si ces derniers ne sont pas sécrétés, il est vraisemblable que les enzymes de la voie de l'ergostérol ont une forte affinité pour les pyrophosphates des terpènes, qui sont ainsi polymérisés essentiellement en ergostérol.

La présente invention repose sur la mise en évidence du fait que certains mutants de S. cerevisiae sont capables de sécréter des terpènes, contrairement à ce qui était admis jusqu'à présent, ces dites souches pouvant être utilisées, en outre, pour la préparation directe de boissons aromatiques fermentées ou non.

Plus particulièrement, la présente invention concerne un procédé d'obtention d'arômes terpéniques, caractérisé en ce qu'on cultive, sur un milieu de culture approprié, un mutant de S. cerevisiae, bloqué dans la voie de synthèse de l'ergostérol, sécrétant des terpènes aromatiques.

On a pu mettre, en effet, en évidence, grâce aux travaux sur lesquels repose la présente invention, le fait que la biosynthèse de l'ergostérol bloquée par des mutations dans certaines étapes pour certaines souches (appelés ci-après mutants auxotrophes pour l'ergostérol) se traduit par des accumulations d'intermédiaires terpéniques. Les mutations affectant la voie des stérols chez la levure S. cerevisiae les plus susceptibles de se traduire par des accumulations de pyrophosphates des terpènes correspondent aux bloquages dans les étapes catalysées par la squalène-synthétase et la farnésyl-diphosphate synthase (EC 2.5.1.10).

Des souches de ce type peuvent être sélectionnées par mutation de souches de Saccharomyces et sélection sur un milieu contenant un stérol, notamment de l'ergostérol, et de la nystatine.

Toutefois, à cause de l'auxotrophie pour l'ergostérol, certaines de ces souches, par exemple la souche 134 qui sera décrite plus complètement dans les exemples, présentent dans certains cas une croissance relativement faible à cause des teneurs généralement peu élevées en stérol des jus de fruits. Afin de pallier les fluctuations qualitatives et quantitatives souvent importantes qui sont observées lorsque l'on fait fermenter ce type de souches, on peut, notamment par sous-clonage, obtenir des souches comportant, en plus des mutations décrites précédemment, une mutation suppresseur qui se traduit par une croissance en l'absence d'ergostérol.

La suppression partielle de l'auxotrophie pour l'ergostérol conduit à la restauration du phénotype sauvage.

Par analyse de tétrade, on a pu établir que :
- la mutation ségrège indépendamment de l'auxotrophie pour l'ergostérol,
- la mutation peut être récupérée des spores recombinantes prototrophes pour l'ergostérol,
- la mutation est récessive.

Malgré leur relative indépendance de l'ergostérol exogène, ces clones produisent toujours des quantités importantes de mono-terpènes.

Ce type de souches peut être sélectionné par sous-clonage à partir des souches précédentes sur milieu dépourvu d'ergostérol, par exemple.

Il convient de remarquer que ces souches synthétisent de façon limitée de l'ergostérol, peuvent croître en absence d'ergostérol, mais que cette mutation n'affecte pas le blocage dans la voie des stérols et dans les descendants erg+ elle ne se manifeste pas phénotypiquement.

Afin d'assurer la stabilité génétique des mutants erg-, ils sont conservés à l'état hétérozygote sous forme de diploïdes erg-/erg+. Avant l'emploi, on récupère les spores erg- après sporulation et dissection d'asques.

Lors de la mise en oeuvre de ce procédé, on s'est aperçu que certaines souches erg- issues, après sporulation, des diploïdes erg-/erg+, n'excrétaient plus des terpènes dans le milieu. Ceci a conduit à la mise en évidence d'une mutation récessive supplémentaire qui, associée au blocage dans la voie des stérols, permet l'excrétion des terpènes.

Ainsi les mutants les plus intéressants selon l'invention doivent porter, de préférence, en plus des mutations précédentes, une mutation désignée ter qui est impliquée, soit dans la perméabilisation de la membrane, soit dans la déphosphorylation du géranyl-pyrophosphate.

Dans les haploïdes recombinants erg+, la mutation ter ne se manifeste pas phénotypiquement. Cependant, les spores erg- issues des diploïdes erg+ ter/erg- ter produisent toujours des terpènes.

La conservation des mutants producteurs de terpènes doit se faire à l'état hétérozygote avec comme parent erg+ une souche portant la mutation ter.

Parmi les mutants préférés selon l'invention, il faut citer les mutants portant les mutations suivantes :
- erg-, blocage dans les étapes de la voie des stérols catalysées par la farnésyl-pyrophosphate synthétase et la squalène synthétase,
- un suppresseur récessif non lié à la mutation erg- qui confère une relative indépendance à l'ergostérol exogène sans affecter le blocage dans la voie des stérols,
- ter, mutation récessive permettant la production de terpènes, sans laquelle le géranyl-pyrophosphate accumulé grâce au blocage erg- n'est pas excrété dans le milieu sous forme de géraniol.

Les souches ainsi obtenues sécrètent des terpènes aromatiques en quantités appréciables mais la société demanderesse a pu mettre en évidence la possibilité d'augmenter encore cette sécrétion.

En effet, les études poursuivies ont montré que le fonctionnement des alcool-déshydrogénases (ADH) dévié dans le sens d'une augmentation du fond d'acétyl-CoA permet d'augmenter l'activité de la voie des stérols par une disponibilité plus grande de l'acétyl-CoA.

En particulier, les mutations suivantes sont intéressantes :
- les mutants ADH I⁻, c'est-à-dire les mutants dépourvus d'isoenzyme fermentaire, qui sont incapables de former de l'éthanol à partir de l'acétaldéhyde et présentent donc une production accrue de l'acétyl-CoA;
- les mutants ADH IIc, c'est-à-dire les mutants constitutifs pour l'isoenzyme oxydative, qui oxydent l'éthanol en acétaldéhyde même en présence de fortes quantités de sucre (condition typique pour les jus et moûts de fruits) alors que la souche sauvage n'oxyde l'éthanol qu'en l'absence de sucre.

La combinaison de ces différentes mutations permet d'obtenir, notamment, des mutants produisant des terpènes en quantité importante, notamment des terpènes d'intérêt aromatique tels que le farnésol et le géraniol.

Ainsi, les mutants particulièrement intéressants sont les mutants auxotrophes pour l 'ergostérol et qui sont ADH IIc, les mutants auxotrophes pour l'ergostérol et qui sont ADH I⁻, enfin les mutants ADH I⁻ et ADH IIc.

Le procédé selon la présente invention peut être mis en oeuvre de façon diverse.

Tout d'abord, il est possible d'utiliser la culture de ce type de mutant pour la préparation d'arômes tels que des arômes de Muscat naturel qui seront purifiés à partir de la culture et qui pourront être utilisés pour l'aromatisation de substrats alimentaires, notamment des boissons.

Dans la plupart des cas, toutefois, les arômes terpéniques seront préparés directement in situ par mise en culture de substrats alimentaires, notamment des liquides alimentaires ou des produits alimentaires fluidifiables, tels que des purées de fruits par exemple. Dans ce cas, ces substrats seront directement fermentés par les souches mutantes de S. cerevisiae qui assureront donc l'aromatisation des substrats.

Dans le cas où le mutant utilisé est un mutant ADH I⁻, c'est-à-dire un mutant ne disposant pas d'alcool-déshydrogénase fermentaire, les produits de fermentation obtenus seront des produits non alcoolisés ou à très bas degré d'alcool.

Bien entendu, dans certains cas il peut être intéressant de faire fermenter par ce type de souche des milieux déjà alcoolisés pour leur conférer certains arômes, dans ce cas on pourra effectuer des fermentations successives, par une levure produisant de l'alcool, puis par des mutants selon la présente invention, ou bien effectuer, lorsque cela est possible et compatible avec la croissance des levures, une cofermentation des deux levures. Ces levures peuvent également être utilisées pour réaliser la prise de mousse, par exemple dans les vins mousseux.

Bien entendu, dans de nombreux cas, la préparation de boissons alcoolisées et aromatisées pourra être réalisée en utilisant un mutant selon l'invention mais qui dispose d'une alcool-déshydrogénase fermentaire (ADH I⁺).

Les souches selon la présente invention étant pour certaines auxotrophes pour l'ergostérol, il sera nécessaire, lorsque le milieu de culture est un milieu purement synthétique, de prévoir l'ajout d'ergostérol afin d'assurer la croissance des levures. Dans le cas de substrats d'origine végétale une telle addition n'est, en général, pas nécessaire compte tenu de l'existence de ces composés à l'état de traces dans les différents extraits végétaux.

Les essais effectués à l'aide des mutants selon la présente invention ont montré qu'il était ainsi possible d'obtenir, par exemple, des jus de fruits non alcoolisés présentant l'arôme caractéristique des Muscats, ou bien des produits alcoolisés de type vin ou mousseux présentant des arômes caractéristiques de Muscat ou de Malvoisie.

Les mutants ADH I⁻ ou ADH IIc sont, quant à eux, particulièrement intéressants dans la production des stérols, notamment de l'ergostérol, et de façon générale de tous les produits impliquant une augmentation de la présence d'acétyl-CoA.

Les exemples ci-après, illustrés par les figures, permettront de mettre en évidence d'autres caractéristiques et avantages de la présente invention.

### EXEMPLE 1

### OBTENTION D'UN MUTANT ADH I⁻

Le mutant FL 100 ADH I⁻ est sélectionné à partir de la souche sauvage haploïde FL 100 (ATCC 28383) par la résistance à l'alcool allylique en présence de glucose. Ce mutant, prototrophe pour l'ergostérol, n'accumule pas d'intermédiaires terpéniques, toutes les étapes de la voie des stérols étant fonctionnelles.

En contrepartie, les disponibilités accrues en acétyl-CoA dues à la mutation ADH I⁻ se traduisent par une augmentation significative de la quantité d'ergostérol synthétisé (tableau 1). Ce type de mutants représente des souches hyperproductrices d'ergostérol.

**Tableau I**

| | Ergostérol (% du poids sec) |
|---|---|
| FL 100 | 0,75 ± 0,06 |
| FL 100 ADH I⁻ | 1,06 ± 0,05 |

### EXEMPLE 2

### OBTENTION D'UN MUTANT AUXOTROPHE POUR L'ERGOSTEROL

On effectue une mutagénèse à l'aide de rayons ultraviolets de la souche sauvage FL 100.

On sélectionne ensuite les souches résistantes à la nystatine en présence d'ergostérol. Parmi les mutants résistants on sélectionne l'un d'entre eux que l'on nomme "mutant erg 9".

L'auxotrophie de ce mutant est confirmée sur un milieu complet (extrait de levure 1 %, bactopeptone 1 %, glucose 2 %) avec ou sans ergostérol (80 µg/ml).

La mesure de l'activité squalène-synthétase s'est effectuée dans la fraction microsomale par la méthode de Agnew et Popjak (Agnew W.S., Popjak G. (1978) J. Bio. Chem. 253, 4566-4573) qui met en évidence le fait que ce mutant erg 9 est dépourvu d'activité squalène-synthétase.

Le mutant erg 9 est caractérisé également par l'absence de l'ADH I (faible production d'éthanol) et par la constitutivité pour l'ADH II. Son phénotype est résumé dans le tableau 2.

**Tableau 2**

| | Croissance | | Squalène synthétase (Activité spécifique) | ADH I (Act sp) | ADH II (Act sp) | Ethanol (g/l) |
|---|---|---|---|---|---|---|
| | YPG+erg | YPG | | | | |
| FL 100 | +++ | +++ | 0,24 | 16,2 | 12,3 | 36,2 |
| erg 9 | ++ | - | 0 | 0 | 10,8(*) | 3,7 |

| | | | | | | |
|---|---|---|---|---|---|---|
| (*) en présence de 90 g/l de glucose | | | | | | |

### EXEMPLE 3

### OBTENTION D'UN MUTANT AUXOTROPHE POUR L'ERGOSTEROL

Ce mutant a été obtenu à partir d'un mutant thermosensible auxotrophe pour la glycine (aux 32 ts) après mutagénèse UV sur milieu à la nystatine. Ce mutant accumule du diméthyl-allyl-pyrophosphate in vitro et ne produit pas de farnésyl-pyrophosphate en présence d'isopentényl-pyrophate (marqué au carbone 14) et de géranyl-pyrophosphate, ce qui indique l'absence de l'activité farnésyl-diphosphate synthase.

Par ailleurs, le mutant 134 présente le phénomène caractéristique des souches erg 9 ; l'allélisme phénotypique indique la présence d'un allèle de erg 9 ; il s'agit donc d'un mutant double farnésyl-diphosphate synthase.

Comme le mutant erg 9, le mutant 134 est ADH I⁻ et ADH II constitutif. Les résultats concernant la sécrétion du géraniol in vivo montrent que la mutation farnésyl-diphosphate synthase est bradytrophe.

### EXEMPLE 4

Les deux mutants erg 9 et 134 sont mis en culture agitée (24 heures) ou stationnaire (4 jours) en milieu complet (extrait de levure 1 %, bactopeptone 1 %, glucose 2 %, ergostérol 0,008 %) à 28°C. Ces deux mutants sécrètent effectivement du farsénol et du géraniol comme cela est résumé dans le tableau 3.

**Tableau 3**

| | Farnésol (µg/100 ml) | | Géraniol (µg/100 ml) | |
|---|---|---|---|---|
| | Stationnaire | Agité | Stationnaire | Agité |
| FL1 00 | n.d. | n.d. | n.d. | n.d. |
| erg 9 | 24 | 138 | n.d. | n.d. |
| 134 | 17 | 40 | - | 22 |
| n.d. = non détectable | | | | |

A titre comparatif, la souche sauvage FL 100 ne produit ni farnésol ni géraniol, aussi bien en culture agitée qu'en culture stationnaire.

### EXEMPLE 5

### AROMATISATION DE JUS DE FRUITS AVEC LES MUTANTS erg 9 ET 134

Des concentrés de fruits (raisin blanc, raisin rouge, framboise, pêche, poire, banane) dilués à raison de 120 g/l de sucre sont ensemencés avec 10⁷ cpm et incubés sous agitation (150 tours/minute) à 30°C.

L'analyse sensorielle est effectuée au bout de 4 jours sur les jus débarrassés de cellules. Les analyses olfactives indiquent systématiquement une note terpénique dans le cas des mutants erg 9 et 134 à la différence des produits obtenus à l'aide de la souche FL 100 sauvage.

Les résultats observés sont rassemblés dans le tableau 4.

**Tableau 4**

| | FL 100 | Erg 9 | 134 |
|---|---|---|---|
| Raisin blanc | vineux | type muscat | type muscat |
| Raisin rouge | vineux | parfum floral | parfum floral |
| Pêche | "levure" | parfum floral | fruité |
| Poire | "levure" | fruité, floral | fruité, floral |
| Banane | "levure" | note muscat, "farnésol" | note muscat |
| Framboise | "levure, solvant | note très "farnésol" | note vinique |

### EXEMPLE 6

A partir de la souche 134 et par sous-clonage, on obtient une nouvelle souche capable de pousser sur milieu en l'absence d'ergostérol exogène et qui présente les caractéristiques suivantes :
- croissance relativement bonne sans ergostérol (niveau intermédiaire entre 134 et la souche sauvage FL 100),
- biosynthèse d'ergostérol (10 % de la souche sauvage),
- blocage dans l'étape catalysée par la farnésyl-pyrophosphate synthétase, avec accumulation de géranyl pyrophosphate,
- excrétion dans le milieu de culture de monoterpènes, plus particulièrement géraniol et linalool.

Cette souche est beaucoup plus performante du point de vue industriel et permet d'obtenir des productions stables, tant en quantité qu'en qualité.

## Revendications

1. Procédé d'obtention d'arômes terpéniques, caractérisé en ce qu'on cultive, sur un milieu de culture approprié, un mutant de S. cerevisiae bloqué dans la voie de synthèse de l'ergostérol, lequel sécrète des terpènes aromatiques.

2. Procédé selon la revendication 1, caractérisé en ce que le milieu de culture approprié comporte un liquide alimentaire ou un produit alimentaire fluidifiable.

3. Procédé selon l'une des revendications 1 et 2, caractérisé en ce que le mutant a une très faible activité squalène-synthétase et/ou farnésyl-diphosphate synthase..

4. Procédé selon l'une des revendications 1 à 3, caractérisé en ce que le mutant de S. cerevisiae comporte une mutation-suppresseur qui se traduit par une croissance en l'absence d'ergostérol.

5. Procédé selon l'une des revendications 1 à 4, caractérisé en ce que le mutant est un mutant ter.

6. Procédé selon la revendication 5, caractérisé en ce que la conservation des mutants utilisés dans le procédé est effectuée à l'état hétérozygote avec comme parent erg+ une souche portant la mutation ter.

7. Procédé selon l'une des revendications 1 à 6, caractérisé en ce que le mutant est constitutif pour l'alcool-déshydrogénase oxydative.

8. Procédé selon l'une des revendications 1 à 7, caractérisé en ce que le mutant est dépourvu d'alcool-déshydrogénase fermentaire.

9. Procédé selon l'une des revendications 1 à 7, caractérisé en ce que le mutant est ADH I⁺.

10. Procédé selon l'une des revendications 1 à 9, caractérisé en ce que les arômes terpéniques sont extraits du milieu après culture.

11. Procédé selon l'une des revendications 1 à 10, caractérisé en ce que le liquide alimentaire est choisi parmi les jus de fruits, le lait ou les dérivés du lait et les moûts de céréales.

12. Procédé selon l'une des revendications 1 à 8, 10 et 11, caractérisé en ce qu'on prépare des boissons ou des produits aromatisés sans alcool ou à faible degré alcoolique par fermentation des produits de base avec un mutant de S. cerevisiae ADH I⁻ bloqué dans la voie de biosynthèse de l'ergostérol.

13. Procédé selon la revendication 12, caractérisé en ce que le mutant est ADH IIc.

14. Procédé selon la revendication 13, caractérisé en ce que le mutant de S. cerevisiae est utilisé comme levure de prise de mousse dans l'élaboration de vins mousseux.

## Claims

1. Process for obtaining terpene flavourings, characterized in that an S. cerevisiae mutant, blocked in the pathway of ergosterol synthesis and which secretes aromatic terpenes, is cultured on a suitable culture medium.

2. Process according to Claim 1, characterized in that the suitable culture medium contains a liquid foodstuff or a liquidizable food product.

3. Process according to one of Claims 1 and 2, characterized in that the mutant has a very low squalene synthetase and/or farnesyl-diphosphate synthase activity.

4. Process according to one of Claims 1 to 3, characterized in that the S. cerevisiae mutant contains a suppressive mutation which results in growth in the absence of ergosterol.

5. Process according to one of Claims 1 to 4, characterized in that the mutant is a ter mutant.

6. Process according to Claim 5, characterized in that the storage of the mutants used in the process is accomplished in the heterozygous state with a strain bearing the ter mutation as erg+ parent.

7. Process according to one of Claims 1 to 6, characterized in that the mutant is constitutive for oxidative alcohol dehydrogenase.

8. Process according to one of Claims 1 to 7, characterized in that the mutant is devoid of fermentative alcohol dehydrogenase.

9. Process according to one of Claims 1 to 7, characterized in that the mutant is ADH I⁺.

10. Process according to one of Claims 1 to 9, characterized in that the terpene flavourings are extracted from the medium after culturing.

11. Process according to one of Claims 1 to 10, characterized in that the liquid foodstuff is chosen from fruit juices, milk or milk derivatives and cereal musts.

12. Process according to one of Claims 1 to 8 and 10-11, characterized in that drinks or flavoured products without alcohol or with a low alcohol content are prepared by fermentation of the primary products with an ADH I⁻ S. cerevisiae mutant blocked in the pathway of ergosterol biosynthesis.

13. Process according to Claim 12, characterized in that the mutant is ADH IIc.

14. Process according to Claim 13, characterized in that the S. cerevisiae mutant is used as a yeast for the process of secondary fermentation in the production of sparkling wines.

## Patentansprüche

1. Verfahren zur Gewinnung von terpenischen Aromen,
**dadurch gekennzeichnet, daß**
man in einem geeigneten Kulturmedium eine Mutante von S. cerevisiae kultiviert, die in dem Synthesepfad des Ergosterols blockiert ist, welches aromatische Terpene absondert.

2. Verfahren nach Anspruch 1,
**dadurch gekennzeichnet, daß**
das geeignete Kulturmedium eine Nahrungsflüssigkeit oder ein verflüssigbares Nahrungsprodukt enthält.

3. Verfahren nach einem der Ansprüche 1 und 2,
**dadurch gekennzeichnet, daß**
die Mutante eine sehr schwache Squalen-Synthetase-Aktivität und/oder Farnesyl-Diphosphat Synthase aufweist.

4. Verfahren nach einem der Ansprüche 1 bis 3,
**dadurch gekennzeichnet, daß**
die Mutante von S. cerevisiae einen Mutationsunterdrücker aufweist, welcher sich in Abwesenheit von Ergosterol durch Wachstum ausdrückt.

5. Verfahren nach einem der Ansprüche 1 bis 4,
**dadurch gekennzeichnet, daß**
die Mutante eine ter-Mutante ist.

6. Verfahren nach Anspruch 5,
**dadurch gekennzeichnet, daß**
die Konservierung der in dem Verfahren verwendeten Mutanten im heterozygotischen Zustand mit einem die ter-Mutation tragenden Stamm als erg+Erzeuger durchgeführt wird.

7. Verfahren nach einem der Ansprüche 1 bis 6,
**dadurch gekennzeichnet, daß**
die Mutante ein Konstitutiv für die oxydative Alkohol-Dehydrase ist.

8. Verfahren nach einem der Ansprüche 1 bis 7,
**dadurch gekennzeichnet, daß**
die Mutante frei von fermentierender Alkohol-Dehydrase ist.

9. Verfahren nach einem der Ansprüche 1 bis 7,
**dadurch gekennzeichnet, daß**
die Mutante ADH I⁺ ist.

10. Verfahren nach einem der Ansprüche 1 bis 9,
**dadurch gekennzeichnet, daß**
die terpenischen Aromen nach der Kultur aus dem Medium extrahiert werden.

11. Verfahren nach einem der Ansprüche 1 bis 10,
**dadurch gekennzeichnet, daß**
die Nahrungsflüssigkeit aus Fruchtsäften, Milch, Derivaten von Milch oder Stammwürzen ausgewählt wird.

12. Verfahren nach einem der Ansprüche 1 bis 8, 10 und 11,
**dadurch gekennzeichnet, daß**
Getränke oder aromatisierte Produkte ohne Alkoholgehalt, oder mit geringem Alkoholgehalt, durch Fermentierung von Basisprodukten mit einer Mutante von S.cerevisiae ADH I⁻ hergestellt werden, welche in dem Biosynthesepfad von Ergosterol blockiert ist.

13. Verfahren nach Anspruch 12,
**dadurch gekennzeichnet, daß**
die Mutante ADH IIc ist.

14. Verfahren nach Anspruch 13,
**dadurch gekennzeichnet, daß**
die Mutante von S.cerevisiae als Hefe für die Schaumbildung bei der Herstellung von Schaumweinen verwendet wird.
